(19) Europäisches Patentamt
European Patent Office
Office européen des brevets

(11) EP 0 596 541 B1

(12) **EUROPEAN PATENT SPECIFICATION**

(45) Date of publication and mention of the grant of the patent:
**15.03.2000 Bulletin 2000/11**

(51) Int. Cl.[7]: **A61K 31/40**, A61K 31/445

(21) Application number: **93120261.8**

(22) Date of filing: **09.11.1988**

(54) **New use of azatricyclo derivatives and pharmaceutical compositions containing them**

Neue Verwendung von Azatricyclo-Derivaten und diese enthaltende Medikamente

Utilisation de dérivés azatricycliques et compositions pharmaceutiques les contenant

(84) Designated Contracting States:
**BE FR IT**

(30) Priority: **09.11.1987 AT 295287**
**17.12.1987 DE 3742805**

(43) Date of publication of application:
**11.05.1994 Bulletin 1994/19**

(62) Document number(s) of the earlier application(s) in accordance with Art. 76 EPC:
**88118669.6 / 0 315 978**

(73) Proprietor: **Novartis AG**
**4058 Basel (CH)**

(72) Inventors:
- **Grassberger, Maximilian**
  **A-1040 Vienna (AT)**
- **Meingasser, Josef Gottfried**
  **A-2380 Perchtoldsdorf (AT)**
- **Stütz, Anton**
  **A-1238 Vienna (AT)**
- **Stütz, Peter**
  **A-1130 Vienna (AT)**

(56) References cited:
**EP-A- 0 184 162**

- **J.ANTIBIOTICS vol. XL, no. 9 , September 1987 pages 1249 - 55**
- **TRANSPLANTATION PROCEEDINGS vol. 19, no. 5 , October 1987 pages 79 - 83**
- **TRANSPLANTATION PROCEEDINGS vol. 19, no. 5 , October 1987 pages 40 - 44**
- **INVESTIGATIVE OPHTHALMOLOGY & VISUAL SCIENCE vol. 29, no. 8 , August 1988 pages 1265 - 71**
- **CLIN.IMMUNOL.IMMUNOPATHOL. vol. 46, no. 1 , 1988 pages 82 - 90**
- **INVEST.OPTHALM.VISUAL SCI. vol. 29 , 1988 page 374**
- **CLIN.IMMUNOL.IMMUNOPATHOL. vol. 51, no. 1 , 1989 pages 110 - 17**
- **TRANSPLANTATION PROCEEDINGS vol. 19, no. 5 , October 1987 pages 84 - 86**
- **J.ANTIBIOTICS vol. XL, no. 9 , September 1987 pages 1256 - 65**
- **A.MANUILA ET AL. 'Dictionnaire Français de médecine et de biologie (Tome II / III)' 1971 , MASSON & CIE , PARIS * page 167 * * page 983 ***
- **C.ZINK 'Pschyrembel Klinisches Wörterbuch' 1986 , W.DE GRUYTER , BERLIN * page 1682 * * page 495 ***
- **'Dorland's Illustrated Medical Dictionary' , W.B.SAUNDERS CO. , PHILADELPHIA * page 477 * * page 1377 ***
- **E.ERWIN 'Langenscheidts Handwörterbuch Französisch' , LANGENSCHEIDT , BERLIN * page 272 * * page 565 ***

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7/3.6

## Description

The invention concerns the use of the compounds of formula I,

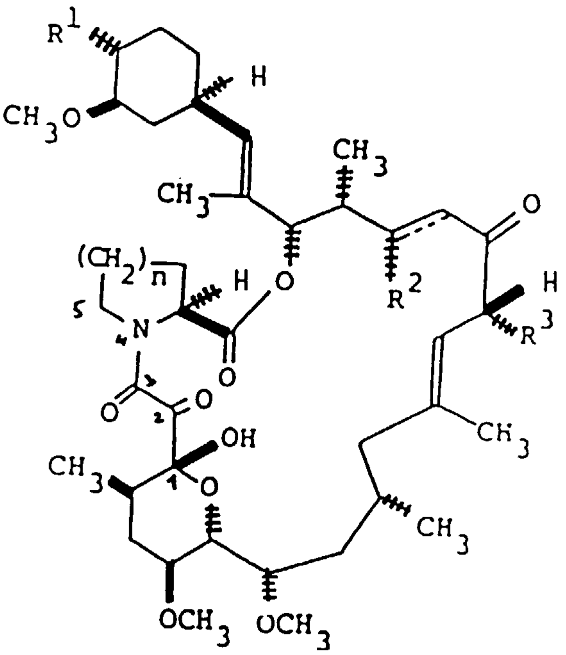

I

wherein

$R^1$ is hydroxy optionally protected by acetyl,
$R^2$ is hydrogen or hydroxy optionally protected by acetyl,
$R^3$ is methyl, ethyl, propyl or allyl,
n is 1 or 2 and
the symbol of a line and dotted line is a single bond or a double bond, in free form or in pharmaceutically acceptable salt form for the preparation of a medicament adapted, with the exception of psoriasis, for topical use in the treatment of inflammatory and hyperproliferative skin diseases and of cutaneous manifestations of immunologically-mediated illnesses, such as: atopic dermatitis, contact dermatitis and further eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus and Alopecia areata.

The compounds of formula I, their preparation and their immunosuppressant and antimicrobial activity are described in e.g. Fujisawa EP 184 162.
It has now been found that the compounds of formula I possess further interesting pharmacological properties which make them indicated for further uses as pharmaceuticals.
It is known and has been repeatedly published in the literature that cyclosporin A (Sandimmun[R]), a highly active immunosuppressant, has practically no activity upon topical administration in e.g. psoriasis (Lancet [1987] p. 806; J. Invest. Dermatol. 90 [1988] 251). In animal testing for contact allergies in mice and guinea pigs cyclosporin A is only active upon topical administration of compositions containing at least 0.1%, and in the pig cyclosporin A is inactive in compositions with up to 5% cyclosporin A.
It has now been found that surprisingly, the compounds of formula I have an excellent topical activity. They are thus very effective in pigs when administered topically against DNFB contact allergies. In mice with oxazolone allergy a superiority by a factor of at least 25 over cyclosporin A is found. Further, the compounds of formula I also exhibit an antiinflammatory effect upon topical administration in animal models of dermatitis caused by irritants. This is indicative of a general antiinflammatory activity upon epicutaneous application. This is corroborated by results from investigations in vitro: inhibition of TPA-induced $PGE_2$ release from macrophages, and inhibition of FMLP- and, respectively, calcium ionophor A 23187-stimulated oxidative burst of human neutrophil polymorphonucleated leukocytes. The compounds of

formula I further exhibit an inhibitory effect in cell culture on the proliferation of human keratinocytes.

The compounds of formula I in free form or in pharmaceutically acceptable salt form are therefore indicated for use upon topical administration in the therapy of inflammatory and hyperproliferative skin diseases and of cutaneous manifestations of immunologically-mediated illnesses, such as: atopic dermatitis, contact dermatitis and further eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus and Alopecia areata.

These activities are apparent in the following test systems:

**1. Determination of the activity after topical administration in models of allergic or allergen-induced contact dermatitis (DTH-reaction)**

1.1. Oxazolone allergy (mouse):

10 µl of a 2% oxazolone solution are applied onto the abdominal skin of mice for sensitization. 8 days later a second exposure with 10 µl of a 2% oxazolone solution is performed by application on the peripheral internal surface of the pinna. 20 minutes and 2 hours after the second exposure has released the challenge reaction, the test solution is applied at the site of the second exposure. Evaluation of the inhibition of inflammation with the test substance is effected by reference to an untreated group treated with the solvent used for dissolving the test substance, alone. 24 hours after the second exposure the animals are killed and the separated pinnae are weighted. The difference in weight between the two pinnae is used for evaluation; the individual differences in the test group and in the solvent control group are statistically compared (by simple variance analysis with subsequent Dunnet test by normal distribution test if normally distributed, otherwise by Kruskal-Wallis' U-test and Wilcoxon-Mann-Whitney's U-test). The activity of the test substance is indicated in %, based on mean values.

Table 1 shows the results obtained in this model for compounds of formula I and cyclosporin A. Ethanol is used as solvent.

Table 1

| Substance | % concentration | % inhibition |
|---|---|---|
| A | 0.13 | 65 |
| | 0.01 | 65 |
| | 0.005 | 52 |
| | 0.002 | 38 |
| | 0.0005 | 35 |
| B | 0.005 | 60 |
| Cyclosporin A | 0.4 | 71 |
| | 0.13 | 56 |
| | 0.04 | 28 |
| | 0.01 | 15 |

1.2. DNFB allergy (swine):

The use of dinitrofluorobenzene (DNFB) or dinitrochlorobenzene (DNCB) for inducing a contact allergy is a classical experimental approach which is also being used in humans (P.S. Friedmann and C. Moss, Models in Dermatology [1987] Maibach, Lowe, Ed., Vol. 2, p. 275-281, Karger-Basel). In view of the resemblance between porcine and human skin a corresponding model for topical testing of substances is built up in the swine. On the 1st and 3rd day 100 µl each of a 10% DNFB preparation is applied onto the inner surface of the right and, respectively, left thigh. On the 14th day each swine is marked on the right and the left side of the back with circular markings of 5 cm in diameter (8 markings per animal) and 150 µl each of a 0.5% DNFB preparation is applied thereon. The substances are tested either in the form of galenical compositions or of a solution. The carriers are used in each case as placebo controls.

The test products are carefully applied 4 times (first 30 minutes, then 6, 24 and 32 hours after release of the challenge reaction). Prior to each application the test areas are evaluated with respect to reddenning, swelling and consistance. The coloration of the test areas is then determined quantitatively with a reflectometer, repeatedly. From the

data on brightness (L*) and saturation (C*) the erythema index is computed according to the following formula: 100 - L* x C* . The mean erythema index is a reflection of the activity according to the following formula:

$$\text{\% inhibition (24, 32, 48, 56 hours)} = 100 - \frac{\text{delta (placebo)} - \text{delta (test)}}{\text{delta (placebo)}}$$

delta = difference with initial value.

The clinical evaluation of the test sites gives clear differences between the sites treated with placebo and with test substance. While sites treated with placebo give areas which are cherry-red, elevated and indurated, with compound A in 5% preparation (solution in 15% dimethylformamide, 42.5% ethanol and 42.5% propylenglycol) the treated areas can hardly be distinguished from the adjacent normal skin. They only show a slight reddish color. The difference in coloration can be clearly shown with the reflectometer. Dexamethasone shows in this model at the same concentration and in the same preparation only weak activity, for cyclossporin A no activity can be shown at all.

**2. Determination of the activity after topical administration in the irritant-induced dermatitis model (mouse)**

2.1. <u>TPA-induced dermatitis:</u>

Skin irritation with TPA in test animals is a method for testing substances as to their antiinflammatory activity after local application (Maibach, Lowe, Ed. <u>Models in Dermatology</u>, Vol. 3 [1987] p. 86-92, Karger-Basel). NMRI mice are given 10 µl of a TPA solution on the inner and outer side of the right pinna (2x10 µl/mouse = 2 x 0.5 µg TPA/mouse). The left pinnae remain untreated. Treatment is effected 30 min. after irritation, by application of 2 x 10 µl of test solution onto the irritated ear surfaces, as described above. The evaluation of the test group is performed by comparison with a group where the right pinna has been treated with only the irritating solution and with the solvent used for the test substance. 6 hours after application of the irritant the animals are killed, the pinnae separated and weighted. The difference in weight of the two pinnae is used for the evaluation, whereby the individual differences of the test groups are statistically compared with the individual differences of the control groups (as under 1.1.). The activity of the test substances is indicated in % on the basis of the average values.

The results compared with indomethacin are reflected in Table 2. The solvent used is a mixture of dimethylacetamide, acetone and ethanol (2/4/4):

Table 2

| Substance | % concentration | % inhibition |
|---|---|---|
| A | 3.6 | 56 |
| | 1.2 | 52 |
| Indomethacin | 3.6 | 31 |
| | 1.2 | 26 |

2.2. <u>Dermatitis induced by croton oil</u>

Croton oil is often used, as TPA, in order to induce an irritant-induced dermatitis on which substances can be tested for their anti-inflammatory activity (Maibach, Love, Ed., <u>Models in Dermatology</u>, Vol. 3 [1987] p. 86-92, Karger-Basel). NMRI-mice are given 15 µl of 0.23% croton oil (in a mixture of dimethylacetamide, acetone and ethanol 2/4/4) on the inner side of the right pinna. Treatment is effected simultaneously with the irritation, the test substance being dissolved in the solution of irritant applied at the auricular test site. Evaluation of the test group is performed by comparison of the inflammation with a group receiving only the irritant solution on the pinna. The animals are killed 6 hours after application of the irritant, the pinnae separated and weighted. The difference between the weights of the two individual pinnae is used for evaluation, by statistical comparison of the single differences in the test group with the single differences in the control group (as under 1.1.). The activity of the test substances is indicated in % based on average values.

The results obtained with compound A compared with indomethacin are shown in Table 3:

Table 3

| Substance | % concentration | % inhibition |
|---|---|---|
| A | 3.6 | 85 |
| | 1.2 | 64 |
| | 0.4 | 52 |
| Indomethacin | 3.6 | 96 |
| | 1.2 | 63 |
| | 0.4 | 11 |

**3. Inhibition of the oxidative burst in human polymorphonuclear neutrophil leukocytes (inhibition of FMLP- or, respectively, A 23187-stimulated chemiluminescence):**

Polymorphonuclear leukocytes (PMNL) are prepared from human peripheral blood (M. Schaude et al., Mycoses 31 (5) [1988] 259-267). Stock solutions of the test substances (500 mg/l) are freshly prepared on the day of experiment in 5% DMSO/RPMI 1640. For the determination of the chemiluminescence (CL) with Biolumat LB 9505 the luminescence indicator DMNH is used. The reaction mixture for determination of the CL of PMNL cells consists of 200 µl PMNL suspension ($5 \times 10^6$ cells/ml), 100 µl of the respective test substance dilution or the solvent system as control and 25 µl of DMNH solution ($2.5 \times 10^{-6}$ M). The CL-reaction is started by addition of either 100 µl of the peptide FMLP ($4 \times 10^{-6}$ M) or of the calcium ionophor A 23187 ($4 \times 10^{-6}$ M). The CL reaction is measured at 37° at 20 seconds over a time span of 20 minutes. 3 parameters are used for evaluation of the results: peak intensity of the radiated light, time span up to the peak and surface area under the reaction curve. As minimal inhibiting concentration the concentration of test substance is chosen where a significant inhibition of all 3 parameters can be observed (Table 4).

Table 4

| Substance | MIC (µM) (FMLP) | MIC (µM) (A 23187) |
|---|---|---|
| A | 0.005 | 0.05 |
| B | 0.01 | 0.01 |
| C | 0.5 | 5 |
| D | < 0.5 | 0.5 |
| E | < 0.5 | 0.05 |

**4. Inhibition of macrophage activation (inhibition of TPA-induced $PGE_2$ release)**

Peritoneal exudate cells of NMRI mice pretreated 3 days earlier with 1.5 ml thioglycolate i.p. are harvested by peritoneal lavage, washed with deficient PBS and resuspended in DMEM medium supplemented with 10% FCS. $1 \times 10^6$ cells are transferred to each well of a 24-wells plate, and the cells are left to adhere 4 hours at 37° and 5% $CO_2$. The cells are then washed twice with deficient PBS. The resultant, more than 95% pure macrophage population is stimulated with TPA (20 µl/l hour) in DMEM-medium devoid of FCS. The conditioned media are centrifuged and the $PGE_2$ contents determined using a $^{125}$I-radioimmunotest. $PGE_2$-release inhibition with the test substances is measured as percentage inhibition compared to the controls.

The results are summarized in Table 5:

Table 5

| Substance | % inhibition at 1 µM |
|---|---|
| A | 30 |

Table 5 (continued)

| Substance | % inhibition at 1 µM |
|---|---|
| B | 60 |
| C | 60 |

**5. Inhibition of proliferation of human keratinocytes**

Cultures of human keratinocytes are obtained by trypsination of human foreskin from newborns or obtained as EpiPack from Clonetics Corp. (San Diego). The keratinocyte cultures are grown in culture flasks in a supplemented keratinocyte medium (KGM). The passages 3 to 5 of 80-90% confluent keratinocytes are resuspended in KGM at a concentration of $1x10^5$ cells/ml, and either 0.1 ml each of this cell suspension is added into a 96-wells microtiter plate or 1 ml each of this cell suspension are added into a 24-wells plate in the presence of test substance. The cells are grown for 48 hours at 37° and 5% $CO_2$. $^3$H-thymidine is incorporated during the last 16 hours (microtiter plate, 1 µCi/well), the cells are checked for their morphology, washed thrice with ice-cold, deficient PBS and twice with trichloroacetic acid, solubilized in 100 µl 0.1 N NaOH containing 1% SDS, and the radioactivity is measured. Alternatively, cells from the 24-well plate are trypsinized (trypsin/EDTA), checked for viability by trypan blue exclusion, and triple aliquots are counted in a cell counter.

The result shows that compound A causes a dose-dependent reduction in proliferation in the concentration range from 0.5 to 5 µM (Table 6).

Table 6

| Substance | % inhibition compared to control |
|---|---|
| A 0.5 µM | 57 |
| A 1 µM | 74 |
| A 5 µM | 94 |
| Solvent | 0 |

In European Patent Specification No. 0315978, of which the present specification is a divisional application, the above use is claimed for the treatment of psoriasis, which has therefore been excluded from the scope of the present application.

<u>Abbreviations</u>:

| | |
|---|---|
| DNFB | 2,4-dinitrofluorobenzene |
| DNCB | Dinitrochlorobenzene |
| TPA | 12-0-tetradecanoylphorbol-13-acetate |
| $PGE_2$ | prostaglandin E2 |
| FMLP | N-formyl-L-methionyl-L-leucyl-L-phemylalanine |
| DTH | delayed-type hypersensitivity |
| A 23187 | calcium ionophor |
| DMSO | dimethylsulfoxide |
| DMNH | 7-dimethylaminonaphthalin-1,2-dicarboxylic acid hydrazide |
| PMNL | polymorphonuclear leukocytes |
| CL | chemiluminescence |
| MIC | minimal inhibitory concentration |
| PBS | phosphate-buffered saline |
| FCS | fetal calf serum |
| SDS | sodim dodecyl sulphate. |

Compound A (FK 506):

17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacos-18-ene-2,3,10,16-tetraone

(disclosed on page 32 in EP 184 162) ($R^1$, $R^2$ = OH; $R^3$ = allyl; n = 2; single bond);

Compound B (dihydro-FK 506):

1,14-dihydroxy-12-[2-(4-hydroxy-3-methoxycyclohexyl)-1-methylvinyl]-23,25-dimethoxy-13,19,21,27-tetramethyl-17-propyl-11,28-dioxa-4-azatricyclo[22.3.1.$0^{4,9}$]octacos-18-ene-2,3,10,16-tetraone

(disclosed on page 98 as Example 21 in EP 184 162) ($R^1$, $R^2$ = OH; $R^3$ = n-propyl; n=2; single bond);

Compound C (dehydrated-FK 506):

17-allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.$0^{4,9}$]octacosa-14,18-diene-2,3,10,16-tetraone

(disclosed on page 95 as part of Example 17 in EP 184 162) ($R^1$ = OH; $R^2$ = H; $R^3$ = allyl; n = 2; double bond);

Compound D (diacetyl-FK 506):

14-acetoxy-12-[2-(4-acetoxy-3-methoxycyclohexyl)-1-methyl-vinyl]-17-allyl-1-hydroxy-23,25-dimethoxy-13,19,21,27-tetramethyl-11,28-dioxa-4-azatricyclo[22.3.1.$0^{4,9}$]octacos-18-ene-2,3,10,16-tetraone

(disclosed on page 89 as part of Example 6 in EP 184 162) ($R^1$, $R^2$ = acetoxy; $R^3$ = allyl; n = 2; single bond);

Compound E (monoacetyl-FK 506):

12-[2-(4-acetoxy-3-methoxycyclohexyl)-1-methylvinyl]-17-allyl-1,14-dihydroxy-23,25-dimethoxy-13,19,21,27-tetra-methyl-11,28-dioxa-4-azatricyclo[22.3.1.$0^{4,9}$]octacos-18-ene-2,3,10,16-tetraone

(disclosed on page 88 as Example 5 in EP 184 162) ($R^1$ = acetoxy; $R^2$ = hydroxy; $R^3$ = allyl; n = 2; single bond).

Compound A (FK 506) is a product isolated from nature. It has a definite stereochemical configuration. However, even though it is disclosed in EP 184 162 with extensive characterization data, the formula given on page 32 in EP 184 162 for FK 506 does not indicate any stereochemical configuration. There is further no indication on the precise configuration of any compound specifically disclosed in EP 184 162. Since there are many asymmetry centers the formula on page 32 thus covers many potential compounds, but only one of them correspnds to FK 506. The exact configuration for FK 506 has been published subsequently, e.g. in H. Tanaka et al., J. Am. Chem. Soc. 109 (1987) 5031-5033, T. Kino et al., J. Antibiotics 40 (1987) 1249-1255 and T. Taga et al., Acta Cryst. C43 (1987) 751-753, and appears to be as follows:

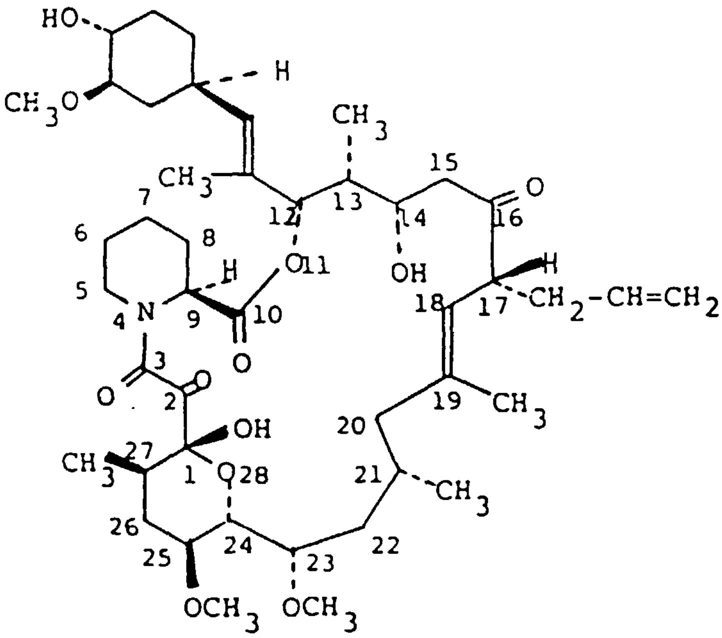

Ia

By implication, since in EP 184 162 the preparation of compounds B, C, D and E is effected starting from FK 506 and using reaction steps which are not modifying the configuration, compounds B, C, D and E also have a configuration corresponding to that shown above for compound A.

An aspect of the invention is thus the use of the compounds of formula I in free form or in pharmaceutically acceptable salt form for the preparation of a medicament adapted, with the psoriasis, for topical use in the treatment of inflammatory and hyperproliferative skin diseases and of cutaneous manifestations of immunologically-mediated illnesses, such as: atopic dermatitis, contact dermatitis and further eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus and Alopecia areata.

Preferred are the compounds of formula I wherein $R^1$, $R^2$ and n are as defined above for formula I, $R^3$ is propyl or allyl and the symbol of a line and dotted line is a single bond; especially preferred is compound A.

For the above use the dosage to be administered is of course dependent on the compound to be administered, the mode of administration and the type of treatment. Satisfactory results are obtained in larger mammals with local administration of a 1-3% concentration of active substance several times daily, e.g. 2 to 5 times daily. Examples of indicated galenical forms are lotions, gels and cremes.

## Claims

**1.** Use of a
compound of formula I

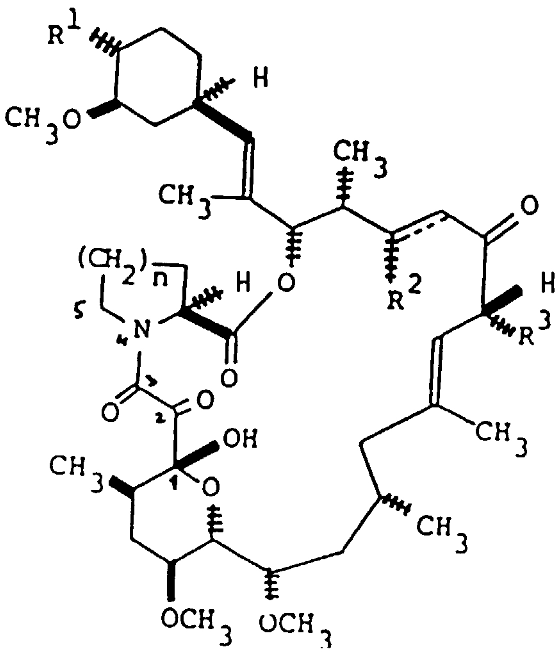

I

wherein

$R^1$ is hydroxy optionally protected by acetyl,
$R^2$ is hydrogen or hydroxy optionally protected by acetyl,
$R^3$ is methyl, ethyl, propyl or allyl,
n is 1 or 2 and
the symbol of a line and dotted line is a single or a double bond, in free form or in pharmaceutically acceptable salt form, for the preparation of a medicament adapted, with the exception of psoriasis, for topical use in the treatment of inflammatory or hyperproliferative skin diseases or of cutaneous manifestations of immunologically-mediated illnesses, atopic dermatitis, contact dermatitis and further eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus or Alopecia areata.

**2.** Use according to claim 1 of a compound of formula I wherein

$R^1$ and $R^2$ are hydroxy,
$R^3$ is allyl,
n is 2 and
the symbol of a line and dotted line is a single bond, in free form or in pharmaceutically acceptable salt form.

**3.** Use according to claim 1 for the preparation of a medicament adapted for topical use in the treatment of conditions as defined in claim 1, comprising a compound of formula I as defined in claim 1, in a 1-3 % concentration of active substance.

**4.** Use according to claim 1 for the preparation of a medicament adapted for topical use in the treatment of conditions as defined in claim 1, which is a lotion, gel or creme.

**5.** Use according to claim 1 for the preparation of a medicament adapted, with the exception of psoriasis, for topical use in the treatment of inflammatory or hyperproliferative skin diseases or of cutaneous manifestations of immunologically-induced illnesses.

**6.** Use according to claim 1 for the preparation of a medicament adapted for topical use in the treatment of atopic dermatitis, contact dermatitis and further eczematous dermatitises, seborrhoeic dermatitis, Lichen planus, Pemphigus,

bullous Pemphigoid, Epidermolysis bullosa, urticaria, angioedemas, vasculitides, erythemas, cutaneous eosinophilias, Lupus erythematosus or Alopecia areata.

7. Use according to claim 6 for the preparation of a medicament adapted for topical use in the treatment of atopic dermatitis.

**Patentansprüche**

1. Verwendung einer Verbindung der Formel I

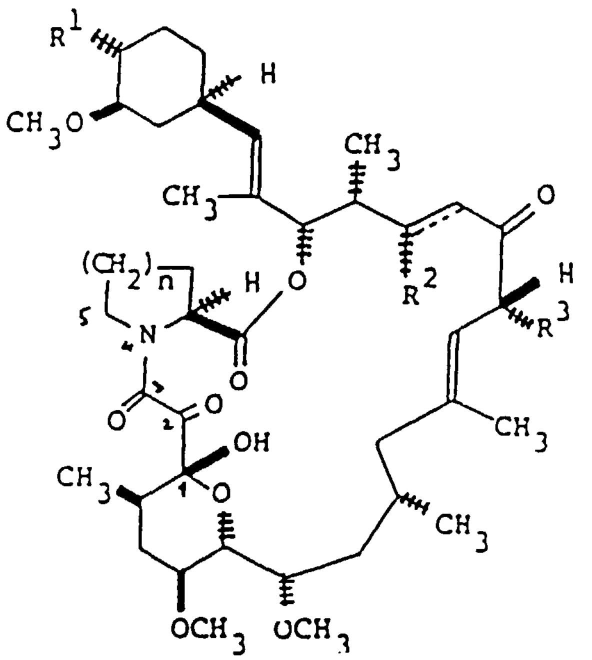

I

worin

$R^1$ für gegebenenfalls durch Acetyl geschütztes Hydroxy steht,
$R^2$ für Wasserstoff oder gegebenenfalls durch Acetyl geschütztes Hydroxy steht,
$R^3$ Methyl, Ethyl, Propyl oder Allyl ist,
n für 1 oder 2 steht und
das Symbol einer Linie oder einer gestrichelten Linie für eine Einfachbindung oder eine Doppelbindung steht, in freier Form oder in Form eines pharmazeutisch annehmbaren Salzes, zur Herstellung eines Arzneimittels, das mit der Ausnahme von Psoriasis formuliert ist für eine topische Anwendung bei der Behandlung entzündlicher oder hyperproliferativer Hauterkrankungen oder kutaner Manifestationen immunmediierter Krankheiten, atopischer Dermatitis, Kontaktdermatitis und weiterer ekzematöser dermatitischer Erkrankungen, seborrhoischer Dermatitis, Lichen planus, Pemphigus, bullösem Pemphigoid, Epidermolysis bullosa, Urtikaria, Angioödem, Vasculitis, Erythema, kutaner Eosinophilie, Lupus erythematosus oder Alopecia areata.

2. Verwendung nach Anspruch 1, worin

$R^1$ und $R^2$ für Hydroxy stehen,
$R^3$ Allyl ist,
n für 2 steht und
das Symbol einer Linie oder einer gestrichelten Linie für eine Einfachbindung oder eine Doppelbindung steht, in freier Form oder in Form eines pharmazeutisch annehmbaren Salzes.

3. Verwendung nach Anspruch 1 zur Herstellung eines Arzneimittels für eine topische Anwendung bei der Behandlung von Zuständen gemäß Definition von Anspruch 1, umfassend eine Verbindung der Formel I mit der in Anspruch 1 angegebenen Definition, in einer Wirkstoffkonzentration von 1 bis 3 %.

4. Verwendung nach Anspruch 1 zur Herstellung eines Arzneimittels für eine topische Anwendung bei der Behandlung von Zuständen gemäß Definition von Anspruch 1, in Form einer Lotion, eines Geles oder einer Creme.

5. Verwendung nach Anspruch 1 zur Herstellung eines Arzneimittels, das mit der Ausnahme von Psoriasis formuliert ist für eine topische Anwendung bei der Behandlung entzündlicher oder hyperproliferativer Hauterkrankungen oder kutaner Manifestationen immuninduzierter Erkrankungen.

6. Verwendung nach Anspruch 1 zur Herstellung eines Arzneimittels für eine topische Anwendung bei der Behandlung von atopischer Dermatitis, Kontaktdermatitis und weiterer ekzematöser dermatischer Erkrankungen, seborrhoischer Dermatitis, Lichen planus, Pemphigus, bullösem Pemphigoid, Epidermolysis bullosa, Urtikaria, Angioödema, Vasculitis, Erythema, kutaner Eosinophilie, Lupus erythematosus oder Alopecia areata.

7. Verwendung nach Anspruch 6 zur Herstellung eines Arzneimittels für eine topische Anwendung bei der Behandlung von atopischer Dermatitis.

**Revendications**

1. Utilisation d'un composé de formule I

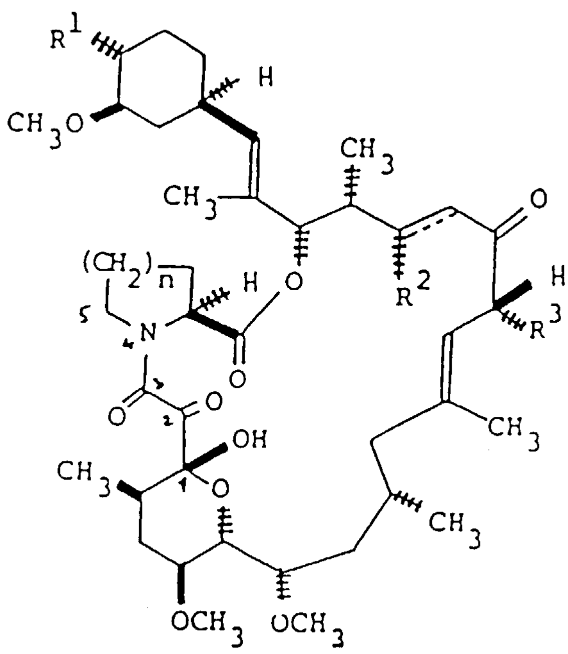

I

où

$R^1$ signifie un groupe hydroxy éventuellement protégé par un groupe acétyle,
$R^2$ signifie l'hydrogène ou un groupe hydroxy éventuellement protégé par un groupe acétyle,
$R^3$ signifie un groupe méthyle, éthyle, propyle ou allyle,
n signifie 1 ou 2, et
le symbole d'une ligne et de pointillés signifie une liaison simple ou double, sous forme libre ou sous forme d'un sel pharmaceutiquement acceptable, pour la préparation d'un médicament adapté, à l'exception du psoriasis, pour l'utilisation topique dans le traitement de maladies de la peau du type inflammatoire ou hyperprolifératif ou des manifestations cutanées de maladies à médiation immunologique, de la dermatite atopique, de la dermatite de contact et d'autres dermatites eczémateuses, de la dermatite séborrhéique, du

lichen plan, du pemphigus, du pemphigoïde bulleux, de l'épidermolyse bulleuse, de l'urticaire, des oedèmes angioneurotiques héréditaires, des vasculites, des érythèmes, des éosinophilies cutanées, du lupus érythémateux ou de l'alopecia areata.

**2.** Utilisation selon la revendication 1 d'un composé de formule I où

$R^1$ et $R^2$ signifient un groupe hydroxy,
$R^3$ signifie un groupe allyle,
n signifie 2, et
le symbole d'une ligne et de pointillés signifie une liaison simple, sous forme libre ou sous forme d'un sel pharmaceutiquement acceptable.

**3.** Utilisation selon la revendication 1, pour la préparation d'un médicament adapté pour l'utilisation topique dans le traitement des conditions telles que définies à la revendication 1, comprenant un composé de formule I tel que défini à la revendication 1, à une concentration de 1-3% de substance active.

**4.** Utilisation selon la revendication 1, pour la préparation d'un médicament adapté pour l'utilisation topique dans le traitement des conditions telles que définies à la revendication 1, qui est une lotion, un gel ou une crème.

**5.** Utilisation selon la revendication 1, pour la préparation d'un médicament adapté, à l'exception du psoriasis, pour l'utilisation topique dans le traitement de maladies de la peau du type inflammatoire ou hyperprolifératif ou des manifestations cutanées de maladies à médiation immunologique.

**6.** Utilisation selon la revendication 1, pour la préparation d'un médicament adapté pour l'utilisation topique dans le traitement de la dermatite atopique, de la dermatite de contact et d'autres dermatites eczémateuses, de la dermatite séborrhéique, du lichen plan, du pemphigus, du pemphigoïde bulleux, de l'épidermolyse bulleuse, de l'urticaire, des oedèmes angioneurotiques héréditaires, des vasculites, des érythèmes, des éosinophilies cutanées, du lupus érythémateux ou de l'alopecia areata.

**7.** Utilisation selon la revendication 6, pour la préparation d'un médicament adapté pour l'utilisation topique dans le traitement de la dermatite atopique.